# EUROPEAN PATENT SPECIFICATION

(11) **EP 2 933 326 B1**
(45) Date of publication and mention of the grant of the patent: **07.04.2021**
(21) Application number: 12890047.9
(22) Date of filing: 14.12.2012
(51) Int. Cl.: C12M 1/10, C12M 1/12, B04B 7/16, B04B 5/06, B04B 3/00, B01D 29/15

(54) **CENTRIFUGAL DYNAMIC FILTERING APPARATUS AND CELL SEPARATION SYSTEM USING SAME**
ZENTRIFUGENVORRICHTUNG FÜR DYNAMISCHE FILTRIERUNG UND ZELLTRENNUNGSVORRICHTUNG DAMIT
APPAREIL DE FILTRATION DYNAMIQUE CENTRIFUGE ET SYSTÈME DE SÉPARATION CELLULAIRE À L'AIDE DE CELUI-CI

(43) Date of publication of application: 21.10.2015
(73) Proprietor: Zheng, Chong, Guangzhou, Guangdong 510665 (CN)
(72) Inventor: Zheng, Chong, Guangzhou, Guangdong 510665 (CN)
(74) Representative: Rhein, Alain
(86) International application number: PCT/CN2012/086694
(87) International publication number: WO 2014/089838

(56) References cited:
- CN-A- 1 276 268
- CN-A- 102 655 895
- CN-Y- 2 676 200
- US-A- 5 431 814
- US-A1- 2011 136 650
- US-B1- 6 773 613

## Description

### FIELD OF THE INVENTION

The present invention relates to biological cell separation field, especially for a centrifugal filtration device and a cell separation system having a microporous membrane filter, and a rapid cell separation method is obtained by using this system.

### BACKGROUND OF THE INVENTION

The best treatment of disease is reconstruction of living tissue and growing tissue which is worn out by old age or disease into new tissue, this treatment is called "Cell Treatment". The cell treatment has a century's history, and is widely used in all fields of tumor treatment, liver treatment and dermabrasion, and it has vast development prospects.

The basic question for cell treatment is separating target cell. In prior art, cell separation is carried out by centrifuges; this separating method not only has fussy operation, but also results in mechanical trauma and pollution to cell, owing to the operation which requires drawing out and putting in cell liquid again and again, and this kind of operation requires very strict laboratory environment, which impacts cell quality, and increases cost for cell separation.

Consequently, the improved device, system and method for cell separation are needed.

### SUMMARY OF THE INVENTION

One purpose of the present invention is to provide a centrifugal filtration device with simple structure and easy operation, this device is capable of separating cells rapidly in a fully sealed system, which decreases cell damage during the separation process, and avoids cell pollution, and the cell separation process could be automatically controlled by the computer.

In order to achieve the above purpose, the present invention provides the following technical solution:
A centrifugal filtration device, for separating living cells, including a spindle, a rotary arm which is connected perpendicularly to the spindle axis to the spindle and capable of rotating as the spindle rotates, and a microporous membrane filter which is mounted on the rotary arm; and the microporous membrane filter includes an inlet, an outlet, a front cavity having the inlet formed thereon, a rear cavity having the outlet formed thereon, and a filter membrane arranged between the front cavity and the rear cavity; the diameter of each filter pore formed in the filter membrane is smaller than that of the cell which needs to be separated; the inlet and the front cavity are arranged on a far end referring to the rotary arm, and the outlet and the rear cavity are arranged on a near end referring to the rotary arm; the inlet is arranged at the top or a sidewall of the front cavity, and the outlet is arranged at the bottom or a sidewall of the rear cavity, the water in the cell suspension, the biological particle and the biomolecules pass through the filter membrane owing to the flowing fluid pressure, and the cells are blocked by the filter membrane and flung from the filter membrane to deposit in the front cavity due to the centrifugal force.

A former document, US 5,431,814, also discloses a rotary filtration apparatus for separating a selected material suspended in a fluid, the apparatus comprising a housing being rotatably mounted on a rotation device which rotates the housing such that a material suspended in the fluid in an input chamber is forced to travel under centripetal force in a radially outward direction from the rotation axis.

The rotation of the housing is achieved by a motor via a complex system of pulleys, supporting frame etc, and, most importantly, the inlet and outlet are arranged near the rotational axis of the filtrating system, which leads to a very different way of operating.

Preferably, the length of the rotary arm is 10-30 cm, the rotation speed of the rotary arm is 500-1500 revolutions per minute, and the centrifugal force produced by the rotary arm is 100-500g.

Preferably, the cross section of the microporous membrane filter is round or square.

Preferably, the diameter of the filter pore formed in the filter membrane is 1-30 µm.

Preferably, the filter membrane is made of polyolefins or polyamides material.

Preferably, the filter membrane is made of polypropylene, mixed cellulose, PE (polyethylene) material or nylon material.

Preferably, the inlet of the microporous membrane filter is connected to a inlet tube, which is connected to a pipe assembly via a rotary joint; and the rotary joint is mounted on a holder which is arranged up over the axis of the rotary arm, a fixed component of the rotary joint is communicated to the pipe assembly, a rotary component of the rotary joint is communicated to the microporous membrane filter via the inlet tube, and the microporous membrane filter is capable of filtering cell suspension continuously while the spindle revolves.

The second purpose of the present invention is to provide a cell separation system having said centrifugal filtration device, especially having a microporous membrane filter, with simple structure and easy operation. This cell separation system provides a fully sealed system for separating cells automatically, which could avoid cell pollution.

In order to achieve the above purpose, the present invention provides the following technical solution:
A cell separation system, characterized by including: a disposable fully sealed piping system and an instrument system; wherein the disposable fully sealed piping system includes a microporous membrane filter, a primary filter, a rotary joint, a disposable syringe, a equilibrium liquid container, a cell suspension container, an enzyme solution container, and a pipe assembly. The microporous membrane filter includes an inlet, an outlet, a front cavity having the inlet formed thereon, a rear cavity having the outlet formed thereon, and a filter membrane arranged between the front cavity and the rear cavity. The inlet is arranged at the top or a sidewall of the front cavity, and the outlet is arranged at the bottom or a sidewall of the rear cavity. The diameter of each filter pore formed in the filter membrane is smaller than that of the cell which needs to be separated. The inlet and the front cavity are arranged further away from the point where the centrifugal force is produced than the outlet and the rear cavity, and the water in the cell suspension, the biological particle and the biomolecules being capable of passing through the filter membrane owing to the flowing fluid pressure, and the cells are blocked by the filter membrane and flung from the filter membrane to deposit in the front cavity due to the centrifugal force. The pipe assembly includes a first pipe, a second pipe, a third pipe, a fourth pipe, a fifth pipe, a sixth pipe and a seventh pipe. The cell suspension container is arranged upside down, whose opening communicates with the first pipe. The primary filter is mounted on the first pipe. The equilibrium liquid container is arranged upside down, whose opening communicates with the second pipe. The second pipe is connected to the first pipe. One end of the third pipe communicates with the junction between the first pipe and the second pipe, and the other end communicates with the disposable syringe. One end of the fourth pipe communicates with the disposable syringe, the other end communicates with a fixed end of the rotary joint. One end of the fifth pipe communicates with a rotary end of the rotary joint, the other end communicates with the inlet tube of the microporous membrane filter. One end of the sixth pipe communicates with the outlet of the microporous membrane filter, the other end communicates with the waste collection tank. One end of the seventh pipe communicates with the junction between the first pipe and the second pipe, and the other end is connected to the enzyme solution container. The instrument system includes a rotary arm assembly, an injection pump, a temperature control unit for equilibrium liquid, a temperature control unit for cell suspension, a vibrator for cell suspension, and an electromagnetic controlling valve. An end of the rotary arm assembly is mounted on the microporous membrane filter, a spindle which drives the rotary arm and a rotation axis of the rotary joint are on a straight line. The disposable syringe is controlled by the injection pump. The temperature control unit for equilibrium liquid is arranged outside the equilibrium liquid container, to heat the equilibrium liquid and control its temperature. The temperature control unit for cell suspension is arranged outside the cell suspension container, to heat the cell suspension and control its temperature. The cell suspension container and the temperature control unit for cell suspension are arranged on the vibrator for cell suspension, which oscillates the cell suspension container automatically with the frequency predetermined by the computer. The electromagnetic controlling valve includes a first controlling valve, a second controlling valve, a third controlling valve, and a fourth controlling valve. The first controlling valve is mounted on the first pipe, and arranged in front of the junction between the first pipe and the second pipe. The second controlling valve is mounted on the second pipe. The third controlling valve is mounted on the fourth pipe, and arranged between the rotary joint and the disposable syringe; and the fourth controlling valve is mounted on the seventh pipe.

Preferably, the diameter of each filter pore formed in the primary filter is larger than that of the target cell.

Preferably, the electromagnetic controlling valve is an electromagnetic pinch valve, to control the opening and closing of the pipe assembly.

Preferably, the filter membrane is hydrophilic membrane.

Preferably, the diameter of the filter pore formed in the filter membrane is 1-30 um.

Preferably, the filter membrane is made of polyolefins or polyamides material.

Preferably, the filter membrane is made of polypropylene, mixed cellulose, PE (Polyethylene) material or nylon material.

The advantage of the present invention is that: the centrifugal filtration device and the cell separation system having said centrifugal filtration device with simple structure and easy operation, are capable of separating cells rapidly in a fully sealed system, which decreases cell damage during the separation process, and avoids cell pollution, the cell separation process could be automatically controlled by the computer, and the cell separation system has low demand in laboratory.

### BRIEF DESCRIPTION OF THE DRAWINGS

Figure 1 is a schematic drawing showing a centrifugal filtration device according to an embodiment of the present invention;
Figure 2 is a schematic lateral drawing showing the centrifugal filtration device according to an embodiment of the present invention;
Figure 3 is a schematic drawing showing the microporous membrane filter of the centrifugal filtration device;
Figure 4 is a schematic drawing showing the rotary joint which is connected to the inlet tube of the microporous membrane filter as shown in figure 1; and
Figure 5 is a schematic drawing showing a cell separation system having the centrifugal filtration device with the microporous membrane filter shown in figure 1.

### DETAILED DESCRIPTION OF ILLUSTRATED EMBODIMENTS

The embodiments of the present invention are disclosed in detail by combining with figures below. All the following are the preferred embodiments of the present invention, which is not the limitation of the protection of the present invention.

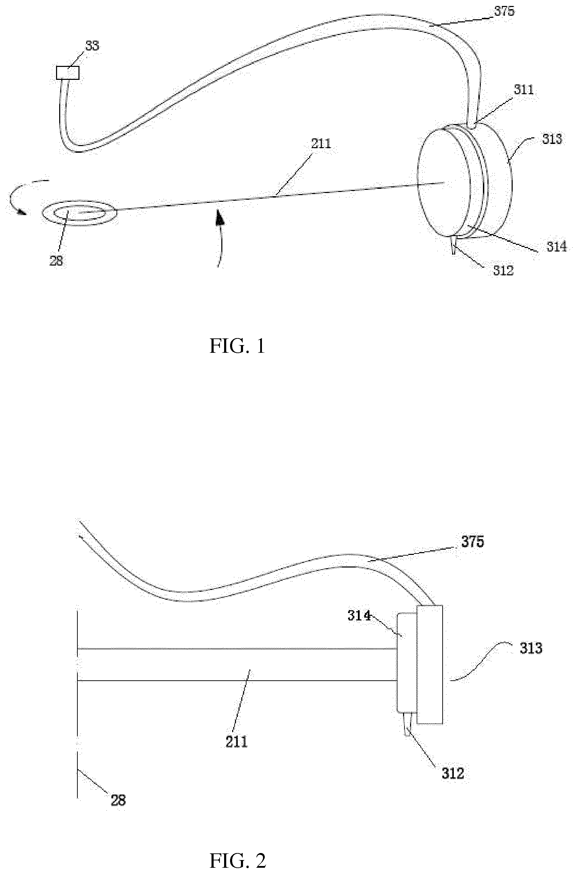

Figures 1, 2 and 3 show the centrifugal filtration device according to an embodiment of the present invention, and the centrifugal filtration device for separating cells includes a spindle 28, a rotary arm 211 which is connected perpendicularly to the spindle axis to the spindle and rotating as the spindle rotates, and a microporous membrane filter 31 which is mounted on a far end referring to the rotary arm.

The microporous membrane filter 31 includes an inlet 311, an outlet 312, a front cavity 313 communicating with the inlet, a rear cavity 314 communicating with the outlet, and a filter membrane 315 arranged between the front cavity 313 and the rear cavity 314.

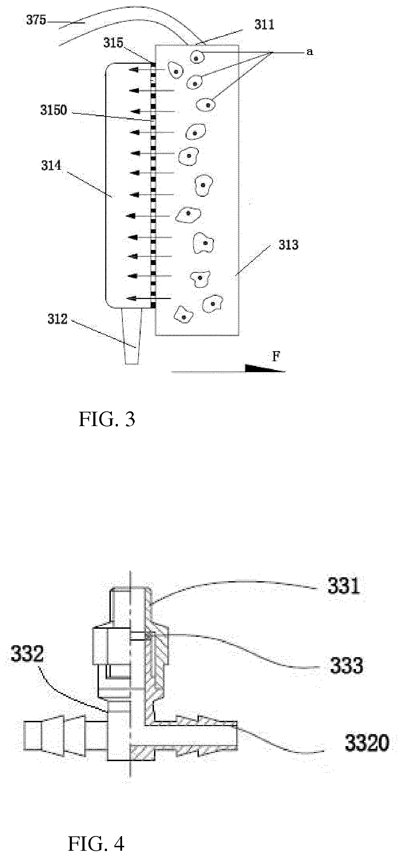

Preferably, the inlet 311 is formed at the top of the front cavity 313, and the outlet 312 is formed at the bottom of the rear cavity 314, so that the liquid could flow due to the flowing fluid pressure produced by the injection, as shown in figure 3.

The filter membrane 315 is hydrophilic membrane, and is made of polyolefins or polyamides material. Preferably, the filter membrane is made of polypropylene, mixed cellulose, PE (polyethylene) material or nylon material. The diameter of the filter pore formed in the filter membrane 315 is smaller than that of the cell which needs to be separated, so that the cells are blocked by the filter membrane and remain in the front cavity 313, and water and biomolecules pass through the filter membrane and run into the rear cavity 314, and then drain out of the outlet 312. In general, the diameter of the cell is 5-30 µm, therefore the diameter of the filter pore of the filter membrane 315 is smaller than 5 µm.

Preferably, the diameter of the filter pore of the filter membrane is 1-30 µm; in an optimum embodiment, the diameter of the filter pore is 3 µm-5 µm.

In this embodiment, the microporous membrane filter 31 has a cavity formed therein whose shape look like a round cake; the surface of the filter membrane which the liquid runs in is arranged on a far end referring to the rotary arm, and the surface of the filter membrane in which the liquid runs out is arranged on a near end referring to the rotary arm. The rotary arm rotates to produce centrifugal force, so that the cells are blocked by the filter membrane and flung from the filter membrane because the cells have bigger size and can withstand more centrifugal force, and the filter pores are kept open, to make the water and useless or harmful biomolecules run through the filter pores under the flowing fluid pressure.

Preferably, the front cavity 313 is arranged on a far end referring to the rotary arm 211, and the rear cavity 314 is arranged on a near end referring to the rotary arm 211, that is, the location of the front cavity 313 is further away from the rotary arm 211 than that of the rear cavity 314. Therefore, the liquid flows from the inlet 311 to the outlet 312, that is the liquid flows from the end far away the rotary arm 211 towards the near end of the rotary arm 211, and this liquid flowing direction is opposite to that of the centrifugal force on the microporous membrane filter 31 while the rotary arm 211 rotates. The cells are blocked by the filter membrane 315 to remain in the front cavity 313 when the cell suspension are in the front cavity 313, and then the cells are flung from the filter membrane 315 owing to the centrifugal force, and the filter pores kept open, to keep the filtering process constant.

In this embodiment, an inlet tube 375 is connected to a pipe assembly via a rotary joint 33. Specifically, as shown in figure 4, the rotary joint 33 includes a fixed component 331 and a rotary component 332, the fixed component 331 has a chamber to receive the rotary component 332 to rotate in the fixed component 331. A seal ring 333 is arranged on the connection between the fixed component 331 and the rotary component 332. The fixed component 331 is fixed and connected to the pipe assembly, and the connecting part 3320 of the rotary component 332 communicates with the inlet tube 375.

Thus, the inlet tube 375 could rotate as the spindle 28 rotates, when the fixed component of the rotary joint is fixed.

Preferably, the rotary joint 33 is arranged on the spindle 28 or its extension cord, so that the inlet tube 375 and the microporous membrane filter 31 rotate synchronously.

The cell separation system according to the present invention is described as follow.

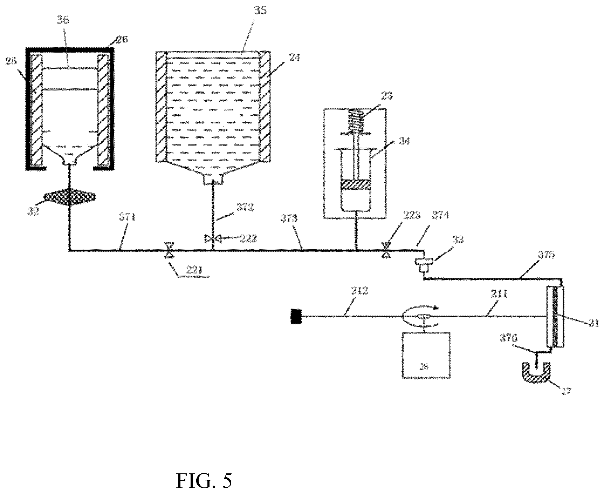

Referring to figure 5, the cell separation system according to the embodiment of the present invention includes two parts, which are a disposable fully sealed piping system and an instrument system.

The disposable fully sealed piping system includes a microporous membrane filter 31, a primary filter 32, a rotary joint 33, a disposable syringe 34, an equilibrium liquid container 35, a cell suspension container 36, an enzyme solution container, and a pipe assembly 37. The detailed description is shown as follow.
(1) The structure of the microporous membrane filter 31 of the centrifugal filtration device has been described above.
(2) the primary filter 32 could filter out impurities in the cell suspension. In this embodiment, the primary filter 32 is arranged in the upstream referring to the liquid flowing direction, that is near the cell suspension container 36, so that some larger particles and some impurities (such as some undigested tissues and big molecule) could be filtered out, during the tissue washing and filtering process.
(3) The structure of the rotary joint 33 of the centrifugal filtration device has been described above.
(4) The disposable syringe 34 is driven by the injection pump 23 (it will be described below), for extracting and injecting liquid.
(5) The equilibrium liquid container 35 is used to hold equilibrium liquid (also called buffer or washing liquid), the equilibrium liquid container 35 is kept under 37°C, controlled by the temperature control unit for equilibrium liquid 24. The temperature of 37°C comes closer to human body temperature, which helps to protect the cells. Also, the temperature control unit for equilibrium liquid 24 could be regulated for temperature in accordance with cell protection requirement.
   The equilibrium liquid could be Phosphate Buffer Saline (PBS) or Lactated Ringer's solution. In this embodiment, the equilibrium liquid is Lactated Ringer's solution preferably, because the electrolyte concentration, PH value and osmotic pressure are very close to those of the extracellular fluids, so as to helps cell survival, washing away the collagenase from the cell liquid , to eliminate the harmful influence on cells. Preferably, the temperature of the raw materials should be kept close to human body temperature during cells extracting process, therefore the present invention provides the temperature control unit for cell suspension 25, which heats the cell suspension container 36 and control it under certain temperature, which is 37°C in general.
(6) The cell suspension container 36 is arranged upside down in this embodiment, whose opening is arranged downward. The cell suspension container 36 is used for containing raw materials from the human body for extracting cells, the raw material could be all kinds of tissues, including but not limiting to: adipose tissue, blood, bone marrow, muscle, skin, liver, muscle membrane, placenta, umbilical cord, body fluids, secretions, and cell culture, etc..
   In this embodiment, the adipose tissue is separated to harvest adipose stem cells. The adipose tissue could be obtained using any suitable process in prior art, such as liposuction (using a syringe) or lipectomy. The amount of extracting adipose tissue depends on various factors, including: capability of extracting adipose tissue and the necessary amount of adipose stem cells. Preferably, in order to mix the collagenase solution with the adipose tissue quickly, and digesting the adipose tissue quickly using the collagenase, the cell suspension container 36 and the temperature control unit for cell suspension 25 are both arranged on the vibrator for cell suspension 26, to oscillate cell suspension container 36.
(7) The enzyme solution container is used for preparing solution during tissue processing, and the enzyme solution is a collagenase solution in this embodiment.
(8) The pipe assembly 371, 372, 372, 373, 374, 375, 376, comprises a first pipe 371, a second pipe 372, a third pipe 373, a fourth pipe 374, a fifth pipe 375, a sixth pipe 376, and a seventh pipe.

The cell suspension container 36 is arranged upside down, whose opening is communicated to the first pipe 371, and the primary filter 32 is mounted on the first pipe 371.

The equilibrium liquid container 35 is arranged upside down, whose opening communicates with the second pipe 372, and the second pipe 372 communicates with the first pipe 371.

One end of the third pipe 373 communicates with the junction between the first pipe 371 and the second pipe 372, and the other end communicates with the disposable syringe 34.

One end of the fourth pipe 374 communicates with the disposable syringe 34, and the other end communicates with a fixed end of the rotary joint 33.

One end of the fifth pipe 375 communicates with a rotary end of the rotary joint 33, and the other end communicates with the inlet of the microporous membrane filter 31.

One end of the sixth pipe 376 communicates with the outlet of the microporous membrane filter 31, and the other end communicates with the waste collection tank 27.

One end of the seventh pipe communicates with the junction between the first pipe and the second pipe, and the other end communicates with the enzyme solution container.

In this system, when the process of cell separation is finished, the fifth pipe 375 which is connected to the inlet and the sixth pipe 376 which is connected to the outlet are cut by a thermal scissors and sealed, so that the microporous membrane filter 31 are sealed to keep the needed cells stored for use.

All pipes described above could be hard or soft, depending on the actual requirement. In this embodiment, all pipes are made of soft material, such as polyethylene pipe which is usually used, silicon resin pipe, or any other material employed by pipes in prior art. The diameter of the pipe depends on the size or number of the tissue and the flowing speed of the liquid, etc. The pipe is capable of sustaining either positive pressure or negative pressure produced by the syringe.

All parts of the disposable fully sealed piping system are for one time use and fully sealed, which guarantees that the process of separating cells from cell suspension is conducted in a sealed pipe system to avoid pollution.

The instrument system which could be reused for the cell separation system is described below.

Referring to figure 5, the instrument system includes a rotary arm, an electromagnetic controlling valve 22, an injection pump 23, a temperature control unit for equilibrium liquid 24, a temperature control unit for cell suspension 25, and a vibrator for cell suspension 26.

An end of a rotary arm 211 of the rotary arm assembly 21 is mounted on the microporous membrane filter 31, a spindle which drives the rotary arm of the rotary arm assembly 21 and a rotation axis of the rotary joint 33 are on a straight line, so that the rotary arm and the rotary joint rotate in synchronism. A balance member 212 with a balance block of the rotary arm assembly 21 is arranged in the opposite end of the rotary arm. The rotary arm assembly 21 is driven by the spindle 28.

The disposable syringe 34 is controlled by the injection pump 23.

The temperature control unit for equilibrium liquid 24 is arranged outside the equilibrium liquid container 35, to heat the equilibrium liquid and control its temperature.

The temperature control unit for cell suspension 25 is arranged outside the cell suspension container 36, to heat the cell suspension and control its temperature. Furthermore, the cell suspension container 36 and the temperature control unit for cell suspension 25 are arranged on the vibrator for cell suspension 26, which oscillates the cell suspension container 36 automatically with the frequency predetermined by the computer.

The electromagnetic controlling valve 22 includes a first controlling valve 221, a second controlling valve 222, a third controlling valve 223, and a fourth controlling valve.

The first controlling valve 221 is mounted on the first pipe 371, and arranged in front of the junction between the first pipe 371 and the second pipe 372.

The second controlling valve 222 is mounted on the second pipe 372.

The third controlling valve 223 is mounted on the fourth pipe 374, and arranged between the rotary joint 33 and the disposable syringe 34.

The fourth controlling valve is mounted on the seventh pipe.

The first controlling valve 221, the second controlling valve 222, the third controlling valve 223, and the fourth controlling valve are all electromagnetic pinch valves.

The disposable fully sealed piping system having the microporous membrane filter 31, the primary filter 32, the rotary joint 33, the disposable syringe 34, the equilibrium liquid container 35, the cell suspension container 36, the enzyme solution container , and the pipe assembly 37, together with the instrument system having the rotary arm 21, the electromagnetic controlling valve 22, the injection pump 23, the temperature control unit for equilibrium liquid 24, the temperature control unit for cell suspension 25, and the vibrator for cell suspension 26, constitutes the above cell separation system, which could complete the process of washing tissue cells, digestion in the enzyme solution, filtering and separating cells, and washing and collecting cells. The cell separation system with simple sealed structure and being adaptable to harvest the required cells, is operated under a nonpolluting environment, and helps in production of cell extraction.

The cell separation system below is in accordance with the first embodiment shown above, and a cell separation method employing this cell separation system is described. A better understanding to the structure and the function of above cell separation system could be obtained, through the description of this cell separation method.

The cell separation method is carried out by extracting adipose stem cells from the adipose tissue from the human body as raw material.

The method includes the following steps (valves which are not mentioned in the steps are closed by default):

### 1. Heating the equilibrium liquid, and connecting the containers for one time use, the filter, syringe and the pipe assembly together.

The certain temperature set in the temperature control unit for equilibrium liquid 24 is 37°C, the equilibrium liquid container 35 is arranged in the temperature control unit for equilibrium liquid 24, to heat the equilibrium liquid until its temperature reaches 37°C, and the equilibrium liquid with 37°C is not only used to prepare collagenase solution, but also provide cell washing liquid. The instrument system is connected to the piping system: the first pipe 371 is connected to the cell suspension container 36, the second pipe 372 is connected to the equilibrium liquid container 35, the seventh pipe is connected to the enzyme solution container , the disposable syringe 34 is connected to the injection pump 23, the rotary joint 33 is mounted on the holder, the microporous membrane filter 31 is mounted on the rotary arm 211.

### 2. Digesting adipose tissue in the enzyme solution.

The adipose tissue is put into the cell suspension container 36, which is not a PVC (polyvinyl chloride) infusion set. The equilibrium liquid whose volume equals to that of the adipose tissue is extracted from the equilibrium liquid container 35, and is poured into the enzyme solution container. In this embodiment, the equilibrium liquid which is Lactated Ringer's solution, is mixed with the collagenase which is taken according to the enzymatic activity described in the collagenase product description, to form the enzyme solution for digesting adipose tissue, and the enzyme solution in the enzyme solution container is poured into the cell suspension container 36. The certain temperature set in the temperature control unit for cell suspension 25 is 37 °C. And then the vibrator for cell suspension 26 which the cell suspension container arranged on starts to oscillate, the speed is 100 RPM (Revolution Per minute), the time is 20-40 minutes, the digestion time is adjusted based on the enzymatic activity and the adipose tissue digestion degree.

The adipose tissue is digested by the enzyme solution to be divided into three layers from bottom to top
- water solution layer,
- emulsion layer,
- and oil layer.

The adipose stem cells are arranged in the water solution layer and the emulsion layer. Since the bottom of the cell suspension container 36 is connected to the first pipe 371, the injection pump is set to filter and separate cells from the cell liquid.

Furthermore, in other embodiment of the present invention, if the raw material is other tissue, the layering of the solution might not be three layers as shown in above embodiment, but the cell liquid amount is under controlled, the person skilled in the art just need to do some adjustments to obtain the targeted cells.

### 3. Filtering out the impurities and molecules using the filter device, separating and extracting cells using the microporous membrane filter.

In step one, the parameters of the filtering process, the centrifuge process and the separation process are set in the computer. The first controlling valve 221 is opened, and the water-soluble cell suspension in the bottom layer in the cell suspension container 36 is drawn out using the disposable syringe 34 which is driven by the injection pump 23. At this point, the cell suspension flows through the primary filter 32, which could filter out the undigested tissue and impurities, etc.

In step two, the first controlling valve 221 is closed, and the third controlling valve 223 is opened, the cell suspension is pushed by the disposable syringe 34 which is driven by the injection pump 23, to run through the fourth pipe 374, the rotary joint 33 and the fifth pipe 375 to pour into the front cavity of the rotating microporous membrane filter 31, and water, smaller biomolecules and collagenase run into the rear cavity through the filter pores, and then run into the waste collection tank 27 through the sixth pipe 376. The cells remain in the front cavity, and flung from the filter membrane owing to the centrifugal force, which avoids membrane fouling. The rotating radius of the rotary arm is 20cm and the rotation speed of the rotary arm is 1500 revolutions per minute.

In step three, the above processes are repeated, until the cell suspension which is under the oil layer in the cell suspension container is draw out completely.

In order to increase efficiency on cell separation, the equilibrium liquid could be poured into the pipe assembly repeatedly and the cell filtering process could be also conducted repeatedly, the steps in detail are as follow: the second controlling valve 222 is opened, the equilibrium liquid with temperature 37°C in the equilibrium liquid container 35 is drawn out, in this embodiment, the equilibrium liquid 100 ml. And then the second controlling valve 222 is closed, and the first controlling valve 221 is opened, and the equilibrium liquid is injected into the cell suspension container 36, and the step two is repeated, so as to obtain cells in the emulsion layer.

### 4. Washing cells in the microporous membrane filter using the equilibrium liquid.

Firstly, the second controlling valve 222 is opened, and the equilibrium liquid in the equilibrium liquid container 35 is drawn out using the disposable syringe 34.

Then, the second controlling valve 222 is closed, the third controlling valve 223 is opened, to make the equilibrium liquid run into the front cavity of the microporous membrane filter 31 through the fourth pipe 374, so as to wash the cell liquid in the front cavity, and remove the harmful small molecules. The washing liquid in this embodiment is 150 ml.

This washing step is carried out to separate cells from enzyme in the cell liquid.

### 5. Removing the microporous membrane filter 31, and sealing it.

The microporous membrane filter 31 is removed, and the cell liquid in it could be used directly. In this embodiment, the inlet tube and the outlet tube are cut by a thermal scissors and sealed, so that the microporous membrane filter stores cells for use. The microporous membrane filter 31 is oscillated by a vibrator just before used.

The cell separation method achieves a series of processes, such as digesting tissue, filtering cells, gaining cells and collecting cells automatically, which are carried out in the disposable fully sealed piping system, avoiding pollution due to exposition to external factors, reducing cell mechanical trauma in the operation, and harvesting cells with high survival rate.

## Claims

1. A centrifugal filtration device, for separating living cells, **characterized by** comprising a spindle (28), a rotary arm (211) which is connected perpendicularly to the spindle axis to the spindle (28) and capable of rotating as the spindle rotates to produce centrifugal force , and a microporous membrane filter (31) which is mounted on the rotary arm (211);
- wherein the microporous membrane filter (31) comprises an inlet (311), an outlet (312), a front cavity (313) having the inlet formed thereon, a rear cavity (314) having the outlet formed thereon, and a filter membrane (315) arranged between the front cavity (313) and the rear cavity (314); the diameter of the filter pore of the filter membrane (315) is 1-30 µm;
- the inlet (311) and the front cavity (313) are arranged on a far end referring to the rotary arm (211), and the outlet (312) and the rear cavity (314) are arranged on a near end referring to the rotary arm (211);
- the inlet (311) is arranged at the top or a sidewall of the front cavity (313), and the outlet (312) is arranged at the bottom or a sidewall of the rear cavity (314).

2. The centrifugal filtration device according to claim 1, **characterized in that** the length of the rotary arm (211) is 10-30 cm, the rotation speed of the rotary arm (211) is 500-1500 revolutions per minute, and the centrifugal force produced by the rotary arm (211) is 100-500 g.

3. The centrifugal filtration device according to claim 1, **characterized in that** the cross section of the microporous membrane filter (31) is round or square.

4. The centrifugal filtration device according to claim 1, **characterized in that** the diameter of the filter pore of the filter membrane (315) is smaller than 5 µm.

5. The centrifugal filtration device according to claim 1, **characterized in that** the filter membrane (315) is made of polyolefin or polyamide material or of mixed cellulose.

6. The centrifugal filtration device according to claim 5, **characterized in that** the filter membrane (315) is made of polypropylene, PE (polyethylene) material or nylon material.

7. The centrifugal filtration device according to claim 1, **characterized in that** the inlet (311) of the microporous membrane filter (31) is connected to an inlet tube (375), which is connected to a piping assembly via a rotary joint (33); and the rotary joint (33) is mounted on a holder which is arranged up over the axis of the rotary arm (211), a fixed component (331) of the rotary joint (33) is communicated to the piping assembly, a rotary component (332) of the rotary joint (33) is communicated to the microporous membrane filter (31) via the inlet tube (375), and the microporous membrane filter (31) is capable of filtering a cell suspension continuously while the spindle revolves.

8. A cell separation system, **characterized by** comprising:
- a disposable fully sealed piping system and an instrument system;
- wherein the disposable fully sealed piping system comprises a microporous membrane filter (31), a primary filter (32), a rotary joint (33), a disposable syringe (34), an equilibrium liquid container (35), a cell suspension container (36), an enzyme solution container, and a pipe assembly (371, 372, 372, 373, 374, 375, 376);
- the microporous membrane filter (31) comprises an inlet (311), an outlet (312), a front cavity (313) having the inlet formed thereon, a rear cavity (314) having the outlet formed thereon, and a filter membrane (315) arranged between the front cavity (313) and the rear cavity (314); the inlet (311) is arranged at the top or a sidewall of the front cavity (313), and the outlet (312) is arranged at the bottom or a sidewall of the rear cavity (314); the diameter of each filter pore formed in the filter membrane (315) is smaller than that of the cell which needs to be separated; the inlet (311) and the front cavity (313) are arranged further away from the point where the centrifugal force is produced than the outlet (312) and the rear cavity (314);
- the pipe assembly (371, 372, 372, 373, 374, 375, 376) comprises a first pipe (371), a second pipe (372), a third pipe (373), a fourth pipe (374), a fifth pipe (375), a sixth pipe (376), and a seventh pipe;
- the cell suspension container (36) is arranged upside down, whose opening is communicated to the first pipe (371); the primary filter (32) is mounted on the first pipe (371);
- the equilibrium liquid container (35) is arranged upside down, whose opening is communicated to the second pipe (372), and the second pipe (372) is connected to the first pipe (371);
- one end of the third pipe (373) is communicated to the junction between the first pipe (371) and the second pipe (372), and the other end is communicated to the disposable syringe (34);
- one end of the fourth pipe (374) is communicated to the disposable syringe (34), the other end is communicated to a fixed end of the rotary joint (33);
- one end of the fifth pipe (375) is communicated to a rotary end of the rotary joint (33), the other end is communicated to the inlet tube of the microporous membrane filter (31);
- one end of the sixth pipe (376) is communicated to the outlet (312) of the microporous membrane filter (31), and the other end is communicated to a waste collection tank (27);
- one end of the seventh pipe is communicated to the junction between the first pipe (371) and the second pipe (372), and the other end is communicated to the enzyme solution container;
- the instrument system comprises a rotary arm assembly (211), an injection pump (23), a temperature control unit for equilibrium liquid (24), a temperature control unit for cell suspension (25), a vibrator for cell suspension (26), and an electromagnetic controlling valve (22);
- an end of the rotary arm assembly (21) is mounted on the microporous membrane filter (31), a spindle (28) which drives the rotary arm (21) and a rotation axis of the rotary joint (33) are on a straight line;
- the disposable syringe (34) is controlled by the injection pump (23); the temperature control unit for equilibrium liquid (24) is arranged outside the equilibrium liquid container (35), to heat the equilibrium liquid (24) and control its temperature; the temperature control unit for cell suspension (25) is arranged outside the cell suspension container (36), to heat the cell suspension (25) and control its temperature; the cell suspension container (36) and the temperature control unit for cell suspension (25) are arranged on the vibrator (26) for cell suspension (25), which oscillates the cell suspension container (36) automatically with the frequency predetermined by a computer;
- the electromagnetic controlling valve (22) comprises a first controlling valve (22), a second controlling valve (222), a third controlling valve (223), and a fourth controlling valve;
- the first controlling valve (221) is mounted on the first pipe (371), and arranged in front of the junction between the first pipe (371) and the second pipe (372);
- the second controlling valve (222) is mounted on the second pipe (372);
- the third controlling valve (223) is mounted on the fourth pipe (374), and arranged between the rotary joint (33) and the disposable syringe (34);
- the fourth controlling valve is mounted on the seventh pipe.

9. The cell separation system according to claim 8, **characterized in that** the diameter of each filter pore formed in the primary filter (32) is larger than that of the target cell.

10. The cell separation system according to claim 8, **characterized in that** the electromagnetic controlling valve (22) is an electromagnetic pinch valve, to control the opening and closing of the pipe assembly (371, 372, 372, 373, 374, 375, 376).

11. The cell separation system according to claim 8, **characterized in that** the filter membrane (315) is hydrophilic membrane.

12. The cell separation system according to claim 8, **characterized in that** the diameter of the filter pore formed in the filter membrane (315) is 1-30 µm.

13. The cell separation system according to claim 10, **characterized in that** the filter membrane (315) is made of polyolefin or polyamide material or of mixed cellulose.

14. The cell separation system according to claim 13, **characterized in that** the filter membrane (315) is made of polypropylene, PE (polyethylene) material or nylon material.

## Patentansprüche

1. Zentrifugenvorrichtung für Filterung zum Trennen von lebenden Zellen, **dadurch gekennzeichnet, dass** sie eine Spindel (28), einen Dreharm (211), der senkrecht zur Spindelachse mit der Spindel (28) verbunden und dazu in der Lage ist, sich zu drehen, wenn sich die Spindel dreht, um eine Zentrifugalkraft zu erzeugen, und einen mikroporösen Membranfilter (31) umfasst, der auf den Dreharm (211) montiert ist;
- wobei der mikroporöse Membranfilter (31) einen Einlass (311), einen Auslass (312), einen vorderen Hohlraum (313), auf dem der Einlass gebildet ist, einen hinteren Hohlraum (314), auf dem der Auslass gebildet ist, und eine Filtermembran (315) umfasst, die zwischen dem vorderen Hohlraum (313) und dem hinteren Hohlraum (314) angeordnet ist; der Durchmesser der Filterpore der Filtermembran (315) 1 - 30 µm ist;
- der Einlass (311) und der vordere Hohlraum (313) auf einem entfernten Ende angeordnet sind und mit dem Dreharm (211) in Bezug stehen, und der Auslass (312) und der hintere Hohlraum (314) auf einem nahen Ende angeordnet sind und mit dem Dreharm (211) in Bezug stehen;
- der Einlass (311) auf der oberen Seite oder einer Seitenwand des vorderen Hohlraums (313) angeordnet ist und der Auslass (312) am Boden oder einer Seitenwand des hinteren Hohlraums (314) angeordnet ist.

2. Zentrifugenvorrichtung für Filterung nach Anspruch 1, **dadurch gekennzeichnet, dass** die Länge des Dreharms (211) 10 - 30 cm ist, die Drehgeschwindigkeit des Dreharms (211) 500 - 1500 Umdrehungen pro Minute ist und die Zentrifugalkraft, erzeugt durch den Dreharm (211) 100 - 500 g ist.

3. Zentrifugenvorrichtung für Filterung nach Anspruch 1, **dadurch gekennzeichnet, dass** der Querschnitt des mikroporösen Membranfilters (31) rund oder quadratisch ist.

4. Zentrifugenvorrichtung für Filterung nach Anspruch 1, **dadurch gekennzeichnet, dass** der Durchmesser der Filterpore der Filtermembran (315) kleiner als 5 µm ist.

5. Zentrifugenvorrichtung für Filterung nach Anspruch 1, **dadurch gekennzeichnet, dass** die Filtermembran (315) aus Polyolefin- oder Polyamidmaterial oder gemischter Zellulose hergestellt ist.

6. Zentrifugenvorrichtung für Filterung nach Anspruch 5, **dadurch gekennzeichnet, dass** die Filtermembran (315) aus Polypropylen-, PE (Polyethylen)-Material oder Nylonmaterial hergestellt ist.

7. Zentrifugenvorrichtung für Filterung nach Anspruch 1, **dadurch gekennzeichnet, dass** der Einlass (311) des mikroporösen Membranfilters (31) mit einem Einlassrohr (375) verbunden ist, das mit einer Rohrbaugruppe mit Hilfe eines Drehgelenks (33) verbunden ist; und das Drehgelenk (33) auf einem Halter montiert ist, der über der Achse des Dreharms (211) angeordnet ist, eine feste Komponente (331) des Drehgelenks (33) mit der Rohrbaugruppe verbunden ist, eine Drehkomponente (332) des Drehgelenks (33) mit dem mikroporösen Membranfilter (31) mit Hilfe des Einlassrohrs (375) verbunden ist, und der mikroporöse Membranfilter (31) dazu in der Lage ist, eine Zellsuspension kontinuierlich zu filtern, während sich die Spindel dreht.

8. Zelltrennungssystem, **dadurch gekennzeichnet, dass** es Folgendes umfasst:
- ein wegwerfbares, vollständig abgedichtetes Rohrsystem und ein Instrumentensystem;
- wobei das wegwerfbare, vollständig abgedichtete Rohrsystem einen mikroporösen Membranfilter (31), einen primären Filter (32), ein Drehgelenk (33), eine wegwerfbare Spritze (34), einen Gleichgewichtsflüssigkeitsbehälter (35) einen Zellsuspensionsbehälter (36), einen Enzymlösungsbehälter und eine Rohrbaugruppe (371, 372, 372, 373, 374, 375, 376) umfasst;
- der mikroporöse Membranfilter (31) einen Einlass (311), einen Auslass (312), einen vorderen Hohlraum (313), auf dem der Einlass gebildet ist, einen hinteren Hohlraum (314), auf dem der Auslass gebildet ist, und eine Filtermembran (315) umfasst, die zwischen dem vorderen Hohlraum (313) und dem hinteren Hohlraum (314) angeordnet ist; der Einlass (311) auf der oberen Seite oder einer Seitenwand des vorderen Hohlraums (313) angeordnet ist und der Auslass (312) am Boden oder einer Seitenwand des hinteren Hohlraums (314) angeordnet ist; der Durchmesser jeder Filterpore, die in der Filtermembran (315) gebildet ist, kleiner als derjenige der Zelle ist, die getrennt werden muss; der Einlass (311) und der vordere Hohlraum (313) weiter weg von dem Punkt, an dem die Zentrifugalkraft erzeugt wird, als der Auslass (312) und der hintere Hohlraum (314) angeordnet sind;
- die Rohrbaugruppe (371, 372, 372, 373, 374, 375, 376) ein erstes Rohr (371), ein zweites Rohr (372), ein drittes Rohr (373), ein viertes Rohr (374), ein fünftes Rohr (375), ein sechstes Rohr (376) und ein siebtes Rohr umfasst;
- der Zellsuspensionsbehälter (36) umgekehrt angeordnet ist, seine Öffnung mit dem ersten Rohr (371) verbunden ist; der primäre Filter (32) auf dem ersten Rohr (371) montiert ist;
- der Gleichgewichtsflüssigkeitsbehälter (35) umgekehrt angeordnet ist, seine Öffnung mit dem zweiten Rohr (372) verbunden ist, und das zweite Rohr (372) mit dem ersten Rohr (371) verbunden ist;
- ein Ende des dritten Rohrs (373) mit der Verbindungsstelle zwischen dem ersten Rohr (371) und dem zweiten Rohr (372) verbunden ist, und das andere Ende mit der wegwerfbaren Spritze (34) verbunden ist;
- ein Ende des vierten Rohrs (374) mit der wegwerfbaren Spritze (34) verbunden ist, das andere Ende an ein festes Ende des Drehgelenks (33) befestigt ist;
- ein Ende des fünften Rohrs (375) mit einem Drehende des Drehgelenks (33) verbunden ist, das andere Ende mit dem Einlassrohr des mikroporösen Membranfilters (31) verbunden ist;
- ein Ende des sechsten Rohrs (376) mit dem Auslass (312) des mikroporösen Membranfilters (31) verbunden ist, und das andere Ende mit einem Abfallsammeltank (27) verbunden ist;
- ein Ende des siebten Rohrs mit der Verbindungsstelle zwischen dem ersten Rohr (371) und dem zweiten Rohr (372) verbunden ist, und das andere Ende mit dem Enzymlösungsbehälter verbunden ist;
- das Instrumentensystem eine Dreharmeinheit (211), eine Injektionspumpe (23), eine Temperatursteuereinheit für Gleichgewichtsflüssigkeit (24), eine Temperatursteuereinheit für Zellsuspension (25), einen Vibrator für Zellsuspension (26) und ein elektromagnetisches Steuerventil (22) umfasst;
- ein Ende der Dreharmeinheit (21) auf dem mikroporösen Membranfilter (31) montiert ist, eine Spindel (28), die den Dreharm (21) antreibt, und dein Drehachse des Drehgelenks (33) auf einer geraden Linie sind;
- die wegwerfbare Spritze (34) von der Injektionspumpe (23) gesteuert wird; die Temperatursteuereinheit für Gleichgewichtsflüssigkeit (24) außerhalb des Gleichgewichtsflüssigkeitsbehälters (35) angeordnet ist, um die Gleichgewichtsflüssigkeit (24) zu erwärmen und ihre Temperatur zu steuern; die Temperatursteuereinheit für Zellsuspension (25) außerhalb des Zellsuspensionsbehälters (36) angeordnet ist, um die Zellsuspension (25) zu erwärmen und ihre Temperatur zu steuern; der Zellsuspensionsbehälter (36) und die Temperatursteuereinheit für Zellsuspension (25) auf dem Vibrator (26) für Zellsuspension (25) angeordnet sind, der den Zellsuspensionsbehälter (36) automatisch mit der von einem Computer vorbestimmten Frequenz oszilliert;
- das elektromagnetische Steuerventil (22) ein erstes Steuerventil (22), ein zweites Steuerventil (222), ein drittes Steuerventil (223) und ein viertes Steuerventil umfasst;
- das erste Steuerventil (221) auf dem ersten Rohr (371) montiert und vor der Verbindungsstelle zwischen dem ersten Rohr (371) und dem zweiten Rohr (372) angeordnet ist;
- das zweite Steuerventil (222) auf dem zweiten Rohr (372) montiert ist;
- das dritte Steuerventil (223) auf dem vierten Rohr (374) montiert und zwischen dem Drehgelenk (33) und der wegwerfbaren Spritze (34) angeordnet ist;
- das vierte Steuerventil auf dem siebten Rohr montiert ist.

9. Zelltrennungssystem nach Anspruch 8, **dadurch gekennzeichnet, dass** der Durchmesser jeder Filterpore, die im primären Filter (32) gebildet ist, größer als derjenige der Zielzelle ist.

10. Zelltrennungssystem nach Anspruch 8, **dadurch gekennzeichnet, dass** das elektromagnetische Steuerventil (22) ein elektromagnetisches Quetschventil ist, um das Öffnen und Schließen die Rohrbaugruppe (371, 372, 372, 373, 374, 375, 376) zu steuern.

11. Zelltrennungssystem nach Anspruch 8, **dadurch gekennzeichnet, dass** die Filtermembran (315) eine hydrophile Membran ist.

12. Zelltrennungssystem nach Anspruch 8, **dadurch gekennzeichnet, dass** der Durchmesser jeder Filterpore, die in der Filtermembran (315) gebildet ist, 1 - 30 µm beträgt.

13. Zelltrennungssystem nach Anspruch 10, **dadurch gekennzeichnet, dass** die Filtermembran (315) aus Polyolefin- oder Polyamidmaterial oder gemischter Zellulose hergestellt ist.

14. Zelltrennungssystem nach Anspruch 13, **dadurch gekennzeichnet, dass** die Filtermembran (315) aus Polypropylen-, PE (Polyethylen)-Material oder Nylonmaterial hergestellt ist.

## Revendications

1. Dispositif de filtration centrifuge, pour la séparation de cellules vivantes, **caractérisé en ce qu'**il comprend une tige (28), un bras rotatif (211) qui est relié perpendiculairement à l'axe de tige à la tige (28) et apte à tourner lorsque la tige tourne de sorte à produire une force centrifuge, et un filtre à membrane microporeuse (31), lequel est monté sur le bras rotatif (211) ;
- dans lequel le filtre à membrane microporeuse (31) comprend une entrée (311), une sortie (312), une cavité avant (313) sur laquelle est formée l'entrée, une cavité arrière (314) sur laquelle est formée la sortie, et un filtre à membrane (315) agencé entre la cavité avant (313) et la cavité arrière (314) ; le diamètre du pore de filtre de la membrane de filtre (315) est de 1 à 30 µm ;
- l'entrée (311) et la cavité avant (313) sont agencées sur une extrémité éloignée par rapport au bras de rotation (211), et la sortie (312) et la cavité arrière (314) sont agencées sur une extrémité proche par rapport au bras rotatif (211) ;
- l'entrée (311) est agencée en haut ou au niveau d'une paroi latérale de la cavité avant (313), et la sortie (312) est agencée en bas ou au niveau d'une paroi latérale de la cavité arrière (314).

2. Dispositif de filtration centrifuge selon la revendication 1, **caractérisé en ce que** la longueur du bras rotatif (211) est de 10 à 30 cm, la vitesse de rotation du bras rotatif (211) est de 500 à 1500 tours par minute, et la force centrifuge produite par le bras de rotation (211) est de 100 à 500 g.

3. Dispositif de filtration centrifuge selon la revendication 1, **caractérisé en ce que** la section transversale du filtre à membrane microporeuse (31) est ronde ou carrée.

4. Dispositif de filtration centrifuge selon la revendication 1, **caractérisé en ce que** le diamètre du pore de filtre de la membrane de filtre (315) est inférieur à 5 µm.

5. Dispositif de filtration centrifuge selon la revendication 1, **caractérisé en ce que** la membrane de filtre (315) est constituée de matériau de polyoléfine ou polyamide ou de cellulose mélangée.

6. Dispositif de filtration centrifuge selon la revendication 5, **caractérisé en ce que** la membrane de filtre (315) est constituée de matériau de polypropylène, PE (polyéthylène) ou de matériau de nylon.

7. Dispositif de filtration centrifuge selon la revendication 1, **caractérisé en ce que** l'entrée (311) du filtre à membrane microporeuse (31) est reliée à un tube d'entrée (375), lequel est relié à un ensemble de tuyauterie par le biais d'un joint rotatif (33) ; et le joint rotatif (33) est monté sur un support qui est agencé au-dessus de l'axe du bras rotatif (211), un composant fixe (331) du joint rotatif (33) est en communication avec l'ensemble de tuyauterie, un composant rotatif (332) du joint rotatif (33) est en communication avec le filtre à membrane microporeuse (31) par le biais du tube d'entrée (375), et le filtre à membrane microporeuse (31) est apte à filtrer une suspension cellulaire en continu alors que la tige tourne.

8. Système de séparation cellulaire, **caractérisé en ce qu'**il comprend :
- un ensemble de tuyauterie complètement étanche jetable et un système d'instrument ;
- dans lequel le système de tuyauterie complètement étanche jetable comprend un filtre à membrane microporeuse (31), un filtre primaire (32), un joint rotatif (33), une seringue jetable (34), un récipient de liquide d'équilibre (35), un récipient de suspension cellulaire (36), un récipient de solution enzymatique, et un ensemble de tuyauterie (371, 372, 372, 373, 374, 375, 376) ;
- le filtre à membrane microporeuse (31) comprend une entrée (311), une sortie (312), une cavité avant (313) sur laquelle est formée l'entrée, une cavité arrière (314) sur laquelle est formée la sortie, et une membrane de filtre (315) agencée entre la cavité avant (313) et la cavité arrière (314) ; l'entrée (311) est agencée en haut ou au niveau d'une paroi latérale de la cavité avant (313), et la sortie (312) est agencée en bas ou au niveau d'une paroi latérale de la cavité arrière (314) ; le diamètre de chaque pore de filtre formé dans la membrane de filtre (315) est inférieur à celui de la cellule qui doit être séparée ; l'entrée (311) et la cavité avant (313) sont agencées plus loin du point où la force centrifuge est produite que la sortie (312) et la cavité arrière (314) ;
- l'ensemble de tuyauterie (371, 372, 372, 373, 374, 375, 376) comprend une première tuyauterie (371), une deuxième tuyauterie (372), une troisième tuyauterie (373), une quatrième tuyauterie (374), une cinquième tuyauterie (375), une sixième tuyauterie (376) et une septième tuyauterie ;
- le récipient de suspension cellulaire (36) dont l'ouverture est en communication avec la première tuyauterie (371) est agencé sens dessus dessous ; le filtre primaire (32) est monté sur la première tuyauterie (371);
- le récipient de liquide d'équilibre (35), dont l'ouverture est en communication avec la seconde tuyauterie (372), est agencé sens dessus dessous, et la deuxième tuyauterie (372) est reliée à la première tuyauterie (371) ;
- une extrémité de la troisième tuyauterie (373) est en communication avec la jonction entre la première tuyauterie (371) et la deuxième tuyauterie (372), et l'autre extrémité est en communication avec la seringue jetable (34) ;
- une extrémité de la quatrième tuyauterie (374) est en communication avec la seringue jetable (34), l'autre extrémité est en communication avec une extrémité fixe du joint rotatif (33) ;
- une extrémité de la cinquième tuyauterie (375) est en communication avec une extrémité rotative du joint rotatif (33), l'autre extrémité est en communication avec le tube d'entrée du filtre à membrane microporeuse (31) ;
- une extrémité de la sixième tuyauterie (376) est en communication avec la sortie (312) du filtre à membrane microporeuse (31), et l'autre extrémité est en communication avec un réservoir de collecte de déchets (27) ;
- une extrémité de la septième tuyauterie est en communication avec la jonction entre la première tuyauterie (371) et la deuxième tuyauterie (372), et l'autre extrémité est en communication avec le récipient de solution enzymatique ;
- le système d'instrument comprend un ensemble bras rotatif (211), une pompe d'injection (23), une unité de régulation de température pour le liquide d'équilibre (24), une unité de régulation de température pour la suspension cellulaire (25), un vibreur pour la suspension cellulaire (26), et un robinet de commande électromagnétique (22) ;
- une extrémité de l'ensemble bras rotatif (21) est montée sur le filtre à membrane microporeuse (31), une tige (28) qui entraîne le bras rotatif (21) et un axe de rotation du joint rotatif (33) sont sur une ligne droite ;
- la seringue jetable (34) est commandée par la pompe d'injection (23) ; l'unité de régulation de température pour le liquide d'équilibre (24) est agencée à l'extérieur du récipient de liquide d'équilibre (35), pour chauffer le liquide d'équilibre (24) et réguler sa température ; l'unité de régulation de température pour la suspension cellulaire (25) est agencée à l'extérieur du récipient de suspension cellulaire (36), pour chauffer la suspension cellulaire (25) et réguler sa température ; le récipient de suspension cellulaire (36) et l'unité de régulation de température pour la suspension cellulaire (25) sont agencés sur le vibreur (26) pour la suspension cellulaire (25), lequel fait osciller le récipient de suspension cellulaire (36) automatiquement avec la fréquence prédéterminée par un ordinateur ;
- le robinet de commande électromagnétique (22) comprend un premier robinet de commande (22), un deuxième robinet de commande (222), un troisième robinet de commande (223) et un quatrième robinet de commande (224) ;
- le premier robinet de commande (221) est monté sur la première tuyauterie (371), et agencé devant la jonction entre la première tuyauterie (371) et la deuxième tuyauterie (372) ;
- le deuxième robinet de commande (222) est monté sur la deuxième tuyauterie (372) ;
- le troisième robinet de commande (223) est monté sur la quatrième tuyauterie (374), et agencé entre le joint rotatif (33) et la seringue jetable (34) ;
- le quatrième robinet de commande est monté sur la septième tuyauterie.

9. Système de séparation cellulaire selon la revendication 8, **caractérisé en ce que** le diamètre de chaque pore de filtre formé dans le filtre primaire (32) est supérieur à celui de la cellule cible.

10. Système de séparation cellulaire selon la revendication 8, **caractérisé en ce que** le robinet de commande électromagnétique (22) est un robinet à manchon déformable électromagnétique, pour commander l'ouverture et la fermeture de l'ensemble de tuyauterie (371, 372, 372, 373, 374, 375, 376).

11. Système de séparation cellulaire selon la revendication 8, **caractérisé en ce que** la membrane de filtre (315) est une membrane hydrophile.

12. Système de séparation cellulaire selon la revendication 8, **caractérisé en ce que** le diamètre du pore de filtre formé dans la membrane de filtre (315) est de 1 à 30 µm.

13. Système de séparation cellulaire selon la revendication 10, **caractérisé en ce que** la membrane de filtre (315) est constituée de matériau de polyoléfine ou polyamide ou de cellulose mélangée.

14. Système de séparation cellulaire selon la revendication 13, **caractérisé en ce que** la membrane de filtre (315) est constituée de matériau de polypropylène, PE (polyéthylène) ou de matériau de nylon.
